# EUROPÄISCHE PATENTSCHRIFT

(11) **EP 2 499 999 B1**
(45) Veröffentlichungstag und Bekanntmachung des Hinweises auf die Patenterteilung: **27.04.2016**
(21) Anmeldenummer: 12153483.8
(22) Anmeldetag: 01.02.2012
(51) Int. Cl.: A61F 2/44

(54) **Wirbelsäulenimpantat mit Ringen**
Spinal implant with rings
Implant vertébral avec anneaux

(30) Priorität: 14.03.2011 DE 102011001251
(43) Veröffentlichungstag der Anmeldung: 19.09.2012
(73) Patentinhaber: Ulrich GmbH & Co. KG, 89081 Ulm (DE)
(72) Erfinder: Winkler, Tobias, 86637 Binswangen (DE)
(74) Vertreter: Hentrich Patentanwälte PartG mbB

(56) Entgegenhaltungen:
- WO-A1-99/59492
- WO-A1-2010/145627
- US-A1- 2009 062 862

## Beschreibung

Die Erfindung betrifft ein Implantat gemäß dem Oberbegriff des Anspruchs 1.

Derartige Implantate werden eingesetzt, um einen oder mehrere Wirbelkörper und deren zugehörige Bandscheiben zu ersetzen, wenn diese degenerativen oder traumatischen Erkrankungen unterliegen.

Da die Bandscheiben die Beweglichkeit der Wirbelsäule ermöglichen, besteht bei einem Implantat zum Wirbelkörperersatz ein Interesse daran, dieses so stabil wie nötig und so beweglich wie möglich zu gestalten.

Ein Implantat, das sich dieser Problematik stellt, ist bereits aus der WO 2010/145 627 bekannt, wobei die an der Endplatte eines Wirbelkörpers anliegende Platte des Implantats auf der dem Implantatteil zuweisenden Seite eine rotationssymmetrische Ausnehmung zeigt, die durch die Seitenwand an einem durch eine Ringnut begrenzten Ringbund des Implantatteil geführt ist. Ein derartiges Implantat lässt verschiedene Winkelstellungen oder Orientierungen der Platte zu, besitzt jedoch den Nachteil, dass es bei Verkippen der Platte gegenüber dem Implantatteil verkanten kann. Dieses Verkanten kann nur durch sehr starken Druck an der Platte auf der entgegengesetzten Seite wieder gelöst werden.

Ein Implantat gemäß dem Oberbegriff des Patentanspruchs 1 ist in der WO99/59492 A1 gezeigt. Hierbei ist ein erster Ring mit dem sich in Längsrichtung zwischen den Wirbeln erstreckenden Implantatteil schwenkbar verbunden. Weiterhin ist mit dem ersten Ring ein weiterer, zweiter Ring schwenkbar verbunden. Zwischen dem Implantatteil und dem ersten Ring, sowie zwischen dem ersten Ring und dem weiteren, zweiten Ring sind großzügig Spalte eingebracht, wobei die jeweiligen Verbindungen durch flache Stege realisiert sind.

Es ist daher die Aufgabe der vorliegenden Erfindung, ein Implantat der eingangs genannten Art so auszubilden, dass eine winkelvariable und stabil geführte Bewegung der Platte gewährleistet ist.

Diese Aufgabe wird durch ein Implantat mit dem Merkmalsbestand gemäß Patentanspruch 1 gelöst. Dabei ist der Endring um eine erste Achse schwenkbar gelagert auf einem auf dem Implantatteil um eine zweite Achse verdrehbar gelagerten Ring, wobei die erste Achse und die zweite Achse nicht parallel zueinander orientiert sind.

Mit dieser Ausführungsform ist der Vorteil verbunden, dass eine stufenlose und unbegrenzte Beweglichkeit und damit eine beste Anpassung an die Endplattengeometrie und den Endplattenwinkel geschaffen ist. Durch die stufenlose Anpassung an den Endplattenwinkel können punktuelle Spitzenlasten und das damit in Verbindung gebrachte Einbrechen in den Wirbelkörper vermieden werden. Dieses polyaxiale Gelenk erhält zumindest teilweise die Beweglichkeit der Wirbelsäule. Außerdem ist eine höhere Präzision bei der Anwendung geschaffen. Im Vergleich zu herkömmlichen, als Wirbelkörperersatz fungierenden, Implantaten besteht das Implantat der Erfindung aus weniger Komponenten. Es können mehrere Winkelstellungen mit einem einzigen Implantat abgedeckt werden können, wobei eine einfache Auswahl des richtigen Implantats, eine einfache Handhabung des Implantats und eine geringere Lagerhaltung gewährleistet ist.

Als vorteilhaft hat sich erwiesen, wenn die erste Achse und/oder die zweite Achse jeweils senkrecht zur Implantatslängsachse orientiert ist, wodurch eine einfache Herstellung der Lagerung geschaffen ist. Hierbei ist es möglich, dass die erste Achse und die zweite Achse unterschiedlich weit von dem der Endplatte zugewandten Ende des Implantats beabstandet sind, sie also axial versetzt angeordnet sind.

Weiterhin ist vorteilhaft, wenn die erste Achse und die zweite Achse derart orientiert sind, unausgelenkt eine Ebene aufzuspannen. Dadurch schneiden sich die erste Achse und die zweite Achse, was eine sichere Anwendung zur Folge hat.

Eine verstärkte Sicherung des Ringes und des Endringes ist durch die vorteilhafte Ausbildung eines Ringbundes am Implantatteil gegeben.

Besonders günstig ist es, wenn der Endring mindestens eine Stiftaufnahme aufweist, zur Aufnahme mindestens eines dem Ring zugeordneten entlang der ersten Achse ausgebildeten Stiftes. Ein Stiftlager ermöglicht eine vereinfachte Montage des Implantats und gewährleistet eine sichere Lagerung.

Es hat sich als förderlich erwiesen, wenn die Stiftaufnahme und der dem Ring zugeordnete Stift zweifach vorgesehen ist, weil dadurch eine stabile und sichere Lagerung des Ringes am Implantatteil geschaffen ist.

Von Vorteil ist weiterhin, wenn der Ring mindestens eine Implantatsstiftaufnahme aufweist zur Aufnahme mindestens eines dem Implantatteil zugeordneten entlang der zweiten Achse ausgebildeten Implantatsstifts. Auch hier ist die Lagerung des Ringes auf dem Implantatteil mittels eines Stiftes sinnvoll, da dadurch ein Verschwenken um die zweite Achse erleichtert ist.

Es ist außerdem sinnvoll, die Implantatsstiftaufnahme und der dem Implantatteil zugeordnete entlang der zweiten Achse ausgebildete Implantatsstift zweifach vorzusehen, damit eine sichere und stabile Lagerung des Ringes am Implantatteil gewährleistet ist.

Eine weitere vorteilhafte Ausführungsform ist dadurch gekennzeichnet, dass der Ring und / oder der Endring auf der dem Wirbelkörper abgewandten Seite mindestens eine axial ausgebildete Aussparung aufweist, wodurch der Ring bzw. der Endring beim Verkippen um die erste Achse bzw. um die zweite Achse erst später am Implantatteil zur Anlage kommt und dadurch ein noch größerer Winkel der Verschwenkung geschaffen ist.

Weiterhin ist günstig, wenn der Ring und / oder der Endring an der dem Implantatteil zugewandten Seite mindestens eine radial ausgebildete Aussparung aufweist, wodurch ebenfalls der Auslenkwinkel des Ringes bzw. des Endringes vergrößert wird.

Als besonders sinnvoll hat sich erwiesen, dass das Implantatteil am Außenumfang eine Ringnut aufweist. Diese Ringnut dient als Anschlag für die Bewegung des Ringes bzw. des Endringes, wodurch der Winkel zu deren Verschwenkung vergrößert ist.

Erfindungsgemäß ist vorgesehen, dass der Ring und / oder der Endring an der dem Implantatteil zugewandten Seite eine konkav ausgebildete Oberfläche aufweist, wodurch der Ring und/oder der Endring sorgfältiger auf dem Implantatteil bzw. dem Ring geführt sind. Diese Gleitführung wird dadurch verbessert, dass erfindungsgemäß der Ring an der dem Implantatteil abgewandten Seite eine konvex ausgebildete Oberfläche aufweist.

Im folgenden wird die Erfindung an in der Zeichnung dargestellten Ausführungsbeispielen näher erläutert; es zeigen:
- Fig. 1: eine perspektivische Ansicht der Erfindung, teils geschnitten gezeigt,
- Fig. 2a - 2c: eine ungeschnitten gezeigte perspektivische Ansicht des Implantats,
- Fig. 3: eine Aufsicht der Erfindung,
- Fig. 4.: eine Seitenansicht des Implantats,
- Fig. 5: eine um 90° um die Implantatslängsachse gedrehte Ansicht von Fig. 4,
- Fig. 6: den Schnitt a-a aus Fig. 3,
- Fig. 7: den Schnitt b-b aus Fig. 4, und
- Fig. 8: den Schnitt c-c aus Fig. 5.

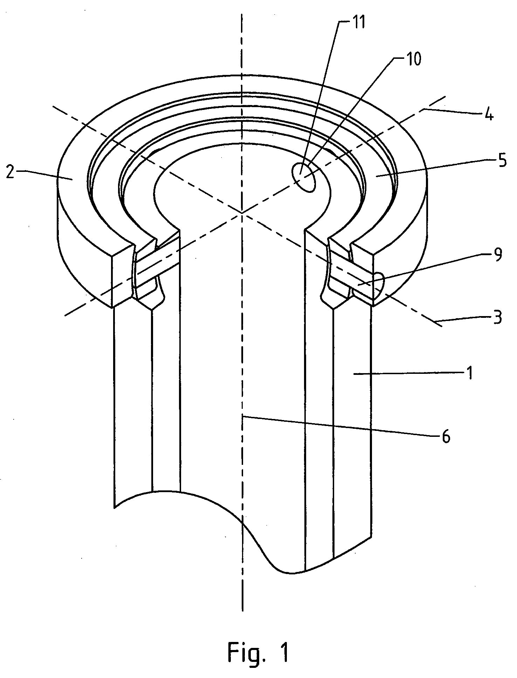

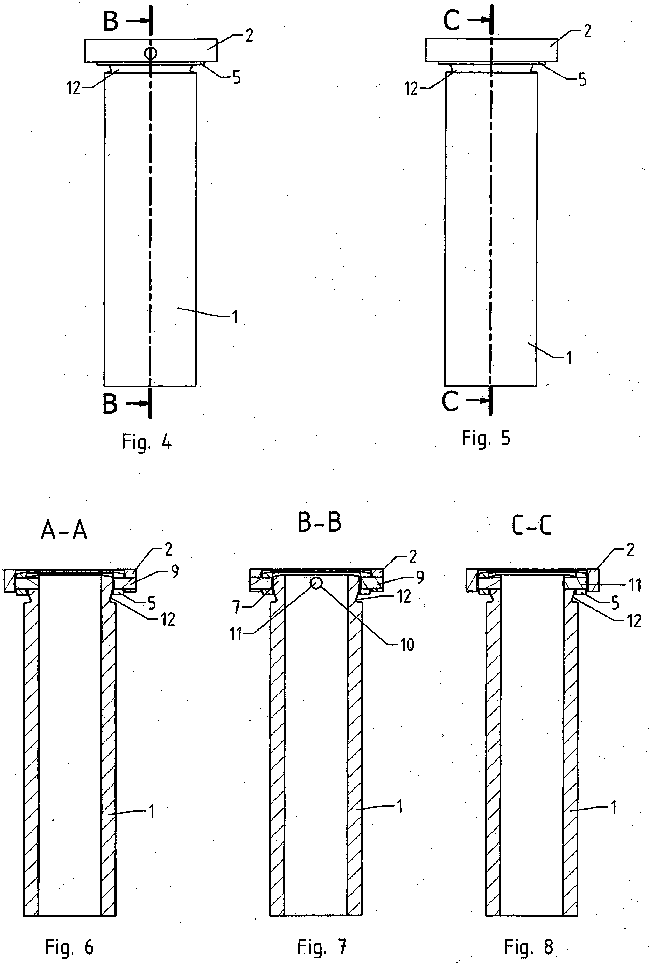

Die Darstellungen der Figur 1 bis Figur 8 zeigen ein Ausführungsbeispiel des erfindungsgemäßen Implantats zum Einsetzen zwischen Wirbelkörper der Wirbelsäule, mit einem Implantatteil 1, das an mindestens einem seiner freien Enden einen Endring 2 zur Anlage an der Oberfläche der Endplatte eines Wirbelkörpers aufweist. Dieses Implantat kann einen oder mehrere Wirbelkörper der Wirbelsäule nach einer traumatischen oder degenerativen Erkrankung ersetzen.

Der Endring 2 ist um eine erste Achse 3 schwenkbar gelagert auf einem auf dem Implantatteil 1 um eine zweite Achse 4 verdrehbar gelagerten Ring 5, wobei die erste Achse 3 und die zweite Achse 4 nicht parallel zueinander orientiert sind.

Durch dieses Gelenk wird die Beweglichkeit der Wirbelsäule erhalten, so dass zum einen eine optimale Anpassung an den Endplattenwinkel des Wirbelkörpers und damit eine optimale Anlage mit reduzierter Flächenpressung erreicht wird, die das Risiko eines Einbrechens in den Wirbelkörper reduziert. Zum anderen kann auch postoperativ eine Anpassung erfolgen, um im Gebrauch durch Mikrobewegungen entstehende Belastungsspitzen zu vermeiden. Dementsprechend ist es mit einer bevorzugten Ausführungsform der vorliegenden Erfindung auch möglich, auf einen Ersatz eines Wirbelkörpers zu verzichten und ausschließlich die zwischen zwei Wirbelkörpern liegende Bandscheibe zu ersetzen. Häufig ist es sinnvoll, das durch den Ring 5 und den Endring 2 gebildete polyaxiale Gelenk an beiden freien Enden des Implantatteils 1 vorzusehen.

Im vorliegenden Ausführungsbeispiel ist die erste Achse 3 orientiert, die zweite Achse 4 zur schneiden und im unausgelenkten Zustand von Ring 5 und Endring 2 spannen die erste Achse und die zweite Achse eine Ebene senkrecht zur Implantatslängsachse 6 auf. Die erste Achse 3 ist zur zweiten Achse 4 senkrecht orientiert.

Im vorliegenden Ausführungsbeispiel weist der Endring 2 zwei Stiftaufnahmen 8 zur Aufnahme von zwei dem Ring 5 zugeordneten entlang der ersten Achse 3 ausgebildeten Stifte 9 auf. Außerdem weist der Ring 5 zwei Implantatsstiftaufnahmen 10 zur Aufnahme von zwei dem Implantatteil 1 zugeordneten entlang der zweiten Achse 4 ausgebildeten Implantatsstifte 11 auf.

Das Implantatteil 1 weist am Außenumfang eine Ringnut 12 auf, wobei die dem Implantatteil 1 zugewandten Oberflächen des Ringes 5 und des Endringes 2 konkav ausgebildet sind. Die Oberfläche des Ringes 5 ist auf der dem Implantatteil 1 abgewandte Seite konvex gebildet.

In Figur 1, die das Implantat perspektivisch und teils geschnitten zeigt, ist deutlich der Stift 9 und der Implantatsstift 11 zu erkennen, die die polyaxiale Ausrichtung des Endrings 2 zur Anpassung an die Endplatte des Wirbelkörpers ermöglichen.

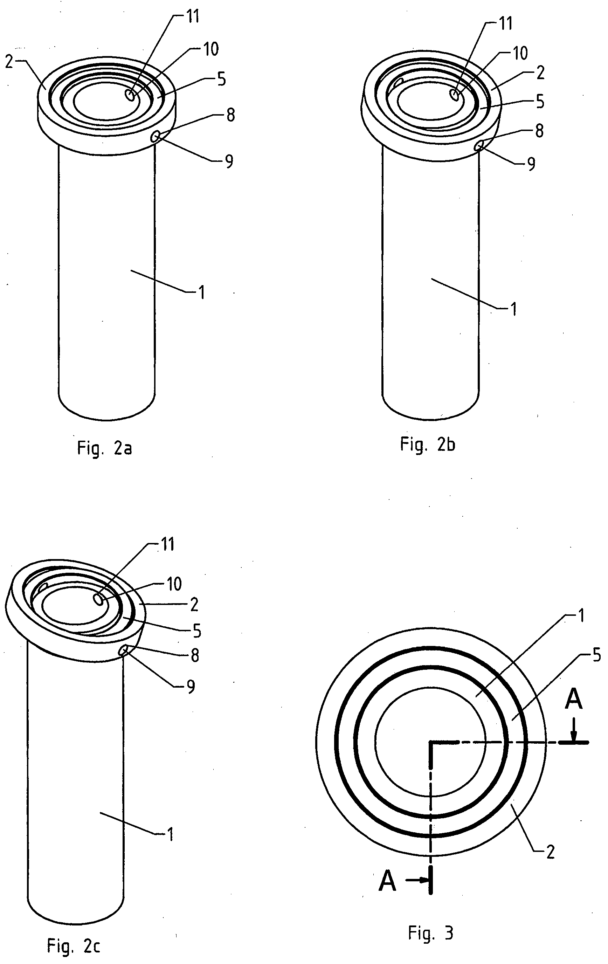

Figur 2a zeigt das Implantat in unausgelenktem Zustand, wobei in Figur 2b das Implantat mit um die zweite Achse 4 verdrehtem Ring 5 dargestellt ist. In Figur 2c ist zudem, ergänzend zu Fig. 2b, der Endring 2 um die erste Achse 3 verschwenkt gezeigt.

### Bezugszeichenliste

- 1: Implantatteil
- 2: Endring
- 3: erste Achse
- 4: zweite Achse
- 5: Ring
- 6: Implantatslängsachse
- 7: Ringbund
- 8: Stiftaufnahme
- 9: Stift
- 10: Implantatsstiftaufnahme
- 11: Implantatsstift
- 12: Ringnut

## Patentansprüche

1. Implantat zum Einsetzen zwischen Wirbelkörper der Wirbelsäule, mit einem Implantatteil (1), das an mindestens einem seiner freien Enden einen Endring (2) aufweist, wobei der Endring (2) um eine erste Achse (3) schwenkbar gelagert ist auf einem auf dem Implantatteil (2) um eine zweite Achse (4) verdrehbar gelagerten Ring (5), wobei die erste Achse (3) und die zweite Achse (4) nicht parallel zueinander orientiert sind, **dadurch gekennzeichnet, dass** der Ring (5) und / oder der Endring (2) an der dem Implantatteil (1) zugewandten Seite eine konkav ausgebildete Oberfläche aufweist, und dass der Ring (5) an der dem Implantatteil (1) abgewandten Seite eine konvex ausgebildete Oberfläche aufweist.

2. Implantat nach einem der Anspruch 1, **dadurch gekennzeichnet, dass** die erste Achse (3) und / oder die zweite Achse (4) im unausgelenkten Zustand senkrecht zur Implantatslängsachse (6) orientiert ist.

3. Implantat nach Anspruch 1 oder 2, **dadurch gekennzeichnet, dass** die erste Achse (3) und die zweite Achse (4) derart orientiert sind, unausgelenkt eine Ebene aufzuspannen.

4. Implantat nach einem der Ansprüche 1 bis 3, **dadurch gekennzeichnet, dass** am Implantatteil (1) ein Ringbund (7) ausgebildet ist.

5. Implantat nach einem der Ansprüche 1 bis 4, **dadurch gekennzeichnet, dass** der Endring (2) mindestens eine Stiftaufnahme (8) aufweist zur Aufnahme mindestens eines dem Ring (5) zugeordneten entlang der ersten Achse (3) ausgebildeten Stiftes (9).

6. Implantat nach Anspruch 5, **dadurch gekennzeichnet, dass** die Stiftaufnahme (8) und der dem Ring (5) zugeordnete Stift (9) zweifach vorgesehen ist.

7. Implantat nach einem der Ansprüche 1 bis 6, **dadurch gekennzeichnet, dass** der Ring (5) mindestens eine Implantatsstiftaufnahme (10) aufweist zur Aufnahme mindestens eines dem Implantatteil (1) zugeordneten entlang der zweiten Achse (4) ausgebildeten Implantatsstiftes (11).

8. Implantat nach Anspruch 7, **dadurch gekennzeichnet, dass** die Implantatsstiftaufnahme (10) und der dem Implantatteil (1) zugeordnete Implantatsstift (11) zweifach vorgesehen ist.

9. Implantat nach einem der Ansprüche 1 bis 8, **dadurch gekennzeichnet, dass** der Ring (5) und / oder der Endring (2) auf der dem Wirbelkörper abgewandten Seite mindestens eine axial ausgebildete Aussparung aufweist.

10. Implantat nach einem der Ansprüche 1 bis 9, **dadurch gekennzeichnet, dass** der Ring (5) und / oder der Endring (2) an der dem Implantatteil (1) zugewandten Seite mindestens eine radial ausgebildete Aussparung aufweist.

11. Implantats nach nach einem der Ansprüche 1 bis 10, **dadurch gekennzeichnet dass** das Implantatteil (1) am Außenumfang eine Ringnut (12) aufweist.

## Claims

1. Implant for insertion between vertebrae of the vertebral column, comprising an implant part (1) which comprises, at least at its free end, an end ring (2), wherein the end ring (2) is mounted such as to rotate about a first axis (3) on a ring (5) which is mounted such as to rotate about a second axis (4), wherein the first axis (3) and the second axis (4) are not oriented parallel to each other, **characterized in that** the ring (5) and/or the end ring (2) comprise a concave configured surface on the side facing the implant part (1), and that the ring (5) comprises a convex configured surface on the side facing away from the implant part (1).

2. Implant according to claim 1, **characterised in that** the first axis (3) and/or the second axis (4) are arranged, in the non-deflected state, oriented perpendicular to the longitudinal axis (6) of the implant.

3. Implant according to claim 1 or 2, **characterised in that** the first axis (3) and the second axis (4) are oriented in such a way that, in the non-deflected state, they span a plane.

4. Implant according to any one of claims 1 to 3, **characterised in that** a ring composite (7) is formed at the implant part (1).

5. Implant according to any one of claims 1 to 4, **characterised in that** the end ring (2) comprises at least one pin mount (8) for accommodating at least one pin (9) allocated to the ring (5) and configured along the first axis (3).

6. Implant according to claim 5, **characterised in that** the pin mount (8) and the pin (9) allocated to the ring (5) are provided as twofold.

7. Implant according to any one of claims 1 to 6, **characterised in that** the ring (5) comprises at least one implant pin mount (10) for accommodating at least one implant pin (11) allocated to the implant part (1) and formed along the second axis (4).

8. Implant according to claim 7, **characterised in that** the implant pin mount (10) and the implant pin (11) allocated to the implant part (1) are provided as twofold.

9. Implant according to any one of claims 1 to 8, **characterised in that** the ring (5) and/or the end ring (2) comprise at least one axially formed cut-out aperture on the side facing away from the vertebra.

10. Implant according to any one of claims 1 to 9, **characterised in that** the ring and/or the end ring (2) comprise at least one radially formed cut-out aperture on the side facing the implant part (1).

11. Implant according to any one of claims 1 to 10, **characterised in that** the implant part (1) comprises an annular groove (12) on its outer circumference.

## Revendications

1. Implant destiné à être inséré entre des corps vertébraux de la colonne vertébrale, comprenant un élément d'implant (1) qui présente une bague d'extrémité (2) à au moins une de ses extrémités libres, la bague d'extrémité (2) étant montée de façon pivotante autour d'un premier axe (3), sur une bague (5) montée sur l'élément d'implant (2) avec possibilité de rotation autour d'un deuxième axe (4), le premier axe (3) et le deuxième axe (4) n'étant pas orientés parallèlement l'un à l'autre, **caractérisé en ce que** la bague (5) et / ou la bague d'extrémité (2) présente(nt), sur le côté tourné vers l'élément d'implant (1), une surface réalisée sous une forme concave, et **en ce que** la bague (5) présente une surface réalisée sous une forme convexe, sur le côté éloigné de l'élément d'implant (1).

2. Implant selon la revendication 1, **caractérisé en ce que**, à l'état non dévié, le premier axe (3) et / ou le deuxième axe (4) est / sont orienté(s) perpendiculairement à l'axe longitudinal d'implant (6).

3. Implant selon la revendication 1 ou 2, **caractérisé en ce que** le premier axe (3) et le deuxième axe (4) sont orientés de manière telle qu'ils définissent un plan lorsqu'ils ne sont pas déviés.

4. Implant selon l'une des revendications 1 à 3, **caractérisé en ce qu'**un collet annulaire (7) est réalisé sur l'élément d'implant (1).

5. Implant selon l'une des revendications 1 à 4, **caractérisé en ce que** la bague d'extrémité (2) présente au moins un logement de tige (8) destiné à recevoir au moins une tige (9) associée à la bague (5) et réalisée le long du premier axe (3).

6. Implant selon la revendication 5, **caractérisé en ce que** le logement de tige (8) et la tige (9) associée à la bague (5) sont prévus deux fois.

7. Implant selon l'une des revendications 1 à 6, **caractérisé en ce que** la bague (5) présente au moins un logement de tige d'implant (10) destiné à recevoir au moins une tige d'implant (11) associée à l'élément d'implant (1) et réalisée le long du deuxième axe (4).

8. Implant selon la revendication 7, **caractérisé en ce que** le logement de tige d'implant (10) et la tige d'implant (11) associée à l'élément d'implant (1) sont prévus deux fois.

9. Implant selon l'une des revendications 1 à 8, **caractérisé en ce que** la bague (5) et / ou la bague d'extrémité (2) présente(nt), sur le côté éloigné du corps vertébral, au moins un évidement réalisé axialement.

10. Implant selon l'une des revendications 1 à 9, **caractérisé en ce que** la bague (5) et / ou la bague d'extrémité (2) présente(nt), sur le côté tourné vers l'élément d'implant (1), au moins un évidement réalisé radicalement.

11. Implant selon l'une des revendications 1 à 10, **caractérisé en ce que** l'élément d'implant (1) présente une rainure annulaire (12) sur le pourtour extérieur.
